(19) Europäisches Patentamt
European Patent Office
Office européen des brevets

(11) Publication number: 0 374 675 A2

(12) # EUROPEAN PATENT APPLICATION

(21) Application number: 89122827.2

(22) Date of filing: 11.12.89

(51) Int. Cl.[5]: C07D 211/90, C07D 211/20, A61K 31/445

(30) Priority: 22.12.88 DK 7160/88

(43) Date of publication of application: 27.06.90 Bulletin 90/26

(84) Designated Contracting States: AT BE CH DE ES FR GB GR IT LI LU NL SE

(71) Applicant: A/S FERROSAN
Sydmarken 5
DK-2860 Soeborg(DK)

(72) Inventor: Lundbeck, Jane Marie
Evas Allé 19
DK-2600 Glostrup(DK)
Inventor: Treppendahl, Svend
Frederiksdalsvej 221
DK-2830 Virum(DK)
Inventor: Jakobsen, Palle
Langkaer Vaene 14
DK-3500 Vaerlose(DK)

(74) Representative: Patentanwälte Grünecker, Kinkeldey, Stockmair & Partner
Maximilianstrasse 58
D-8000 München 22(DE)

(54) **Reduction of piperidine-dion-derivatives and intermediates.**

(57) The invention relates to a novel chemical process for preparing 4-phenylpiperidines having calcium overload blocking activity and antidepressant activity, and to novel intermediates used in that process.

**EP 0 374 675 A2**

## Reduction of piperidine-dion-derivatives and Intermediates

This invention relates to a novel chemical process for preparing 4-phenylpiperidines having calcium overload blocking activity and antidepressant activity, and to novel intermediates used in that process.

Danish patent application no. 5608/87 discloses compounds having the formula (A)

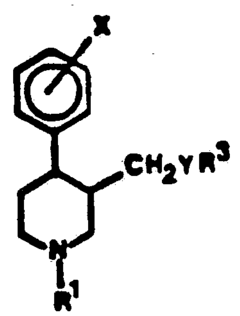

wherein

$R^3$ is 3,4-methylenedioxyphenyl, aryl or heteroaryl which are optionally substituted with one or more $C_{1-6}$-alkyl, $C_{1-6}$-alkoxy, $C_{3-8}$-cycloalkyl, $C_{3-5}$-alkylene or aralkoxy,

$R^1$ is straight or branched $C_{4-8}$-alkyl, $C_{1-8}$-alkoxy-$C_{4-8}$-alkyl, $C_{3-7}$-cycloalkyl, aryloxy-$C_{3-8}$-alkyl, $C_{4-8}$-alkenyl, or $C_{3-8}$-cycloalkylalkyl, or $R^1$ may also be hydrogen or $C_{1-3}$-alkyl, when $R^3$ is aryl, which is substituted with two or more of $C_{1-6}$-alkyl, $C_{1-6}$-alkoxy, $C_{3-8}$-cycloalkyl, aralkoxy, or with $C_{3-5}$-alkylene.

X is hydrogen or halogen, and wherein

Y is O or S

and a salt thereof with a pharmaceutically acceptable acid.

These compounds are disclosed as having calcium overload blocking activity, which makes them useful for the treatment of anoxia, ischemia, migraine and epilepsy.

Danish patent no. 149843 discloses compounds having the formula (B)

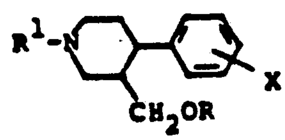

where R is a $C_{1-4}$-alkyl- $C_{2-4}$ alkynyl group, a tetrahydronaphthyl group or a phenyl group which may be sutstituted 1 or 2 times with halogen or $C_{1-4}$-alkyl-, $C_{1-4}$-alkoxy-, $C_{1-4}$-alkylthio-, nitro-, $C_{1-4}$-acylamino- or methylsulphonyl groups or with a methylenedioxy group, $R^1$ is hydrogen, a 2,2,2-trifluoroethyl group or a $C_{1-4}$-alkyl- or $C_{2-4}$-alkynyl group and X is hydrogen, halogen or a hydroxy-, $C_{1-4}$-alkyl-, $C_{1-4}$-alkoxy-, $C_{1-4}$-trifluoroalkyl-, methylthio- or benzyloxy group, or optically active forms thereof or salts of the compounds with pharmaceutically acceptable acids.

These compounds are disclosed as having 5HT uptake inhibiting activity, which makes the compounds useful as antidepressants

The compounds of formula (A) are in the Danish patent application no. 5608/87 prepared by alkylating a compound having the formula (C)

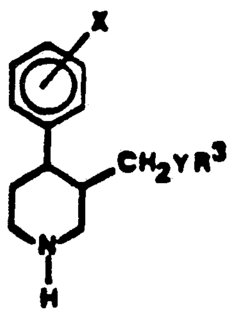

The compounds of Danish patent no. 149843 are made one way or another from an intermediate, having the formula (D)

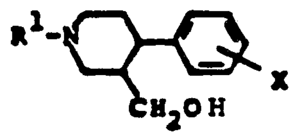

The methods described in above patent application and in above Danish patent all involve intermediates, which preferably should be avoided. One of these intermediates is arecoline, which is a powerful irritant and another intermediate is the compound having the formula (E) 1-methyl-4-phenyl-1,2,3,6-tetrahydro-pyridine (MPTP). This compound is known to be a very powerful neurotoxine and is a strong promotor of irreversible Parkinsonism, (Psychiatry Res., 1 (1979) 249-54; Science, 219 (1983) 979-80).

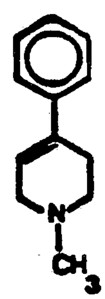

Therefore there is a need to find another process, avoiding this specific compound, for producing the compounds of above Danish patent application no. 5608/87 and Danish patent no. 149843.

The novel process provided with the present invention can be illustrated by the following scheme:

## Scheme 1

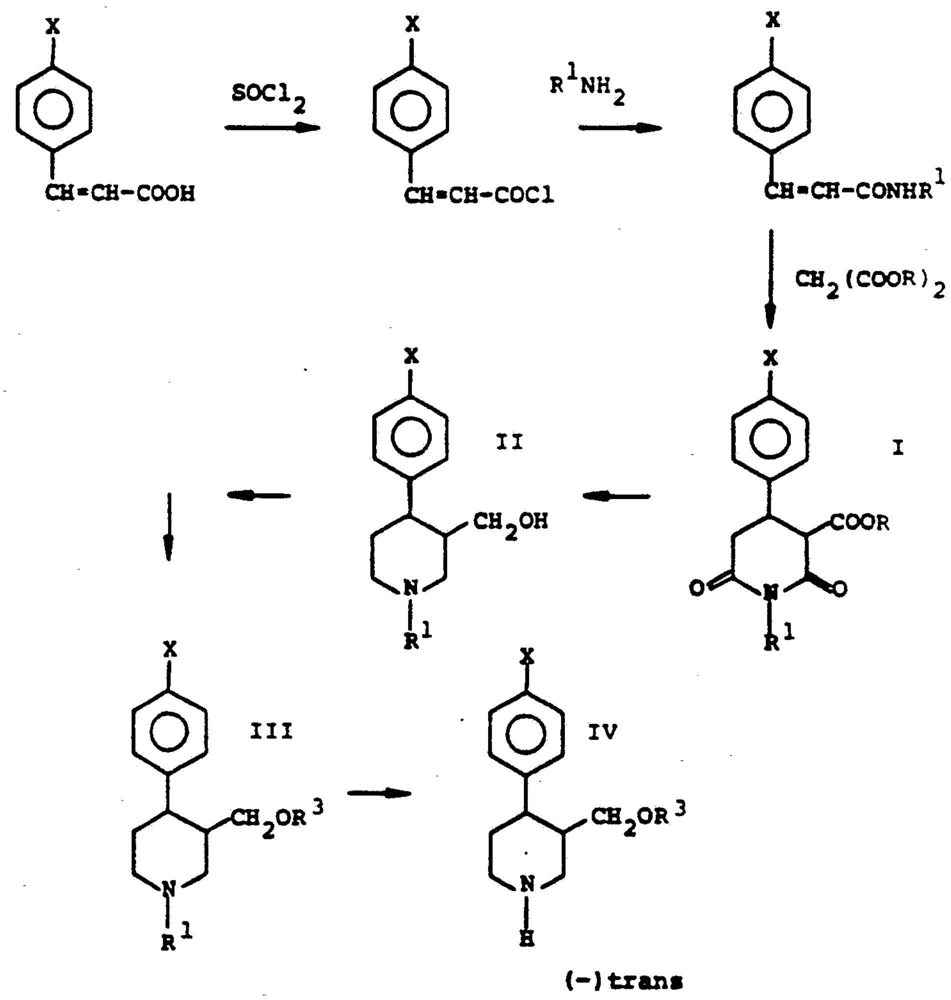


The compounds of formula (I) are useful in the preparation of compounds of formula (II), (III) and (IV). The compounds of formula (III) are examples of those described in Danish patent application no. 5608/87, and compounds having the formula (IV) are examples of the compounds disclosed in Danish patent 149843.

The following examples illustrate the novel process of the present invention, the novel intermediates of the present invention and the utility of the novel intermediates of the present invention.

## EXAMPLE 1

p-fluoro-cinnamoyl chloride

100 ml $SOCl_2$ was dropped to 50.5 g (0.3 mol) p-fluorocinnamic acid, the mixture was subsequently

heated to reflux for 2 h. The excess of $SOCl_2$ was evaporated in vacuo. $CH_2Cl_2$ was added and the reaction mixture was evaporated again. Yield: 56.3 g acid chloride (1).

2-(3,4-methylenedioxyphenyl)-propenoyl chloride was prepared in the same way from 2-(3,4-methylenedioxyphenyl)-propenoic acid and $SOCl_2$. (2).

## EXAMPLE 2

N-pentyl-p-fluorocinnamoyl amide

56.3 g (0.3 mol) (1) was dissolved in 400 ml dry toluene. 150 ml triethylamine was added together with 50 ml (0.43 mol) pentylamine under cooling. After 4 h on ice bath the reaction mixture was allowed to warm to room temp., reaction time 48 h. The two-phase system was cooled on ice which gave 61.85 g white crystals. The remaining oil was poured into ice and washed with $H_2O$. The resulting crystalline compound was filtered off and dried. The first precipitate contained triethylammonium chloride as impurity. Therefore the crystals were washed with water and extracted with ether. The ether layer was dried over $MgSO_4$ and evaporated. The total amount of amide was 68.5 g. M.p. 79.4-7°C. (3).

N-butyl cinnamoyl amide was prepared in exactly the same way from cinnamoyl chloride and butylamine. M.p. 67.4-68.1°C. (4).

N-pentyl cinnamoyl amide was prepared in the same way from cinnamoyl chloride and pentylamine. M.p. 68.1-68.7°C. (5).

N-pentyl-2-(3,4-methylenedioxyphenyl)-propenoyl amide was prepared in the same way from 2-(3,4-methylenedioxyphenyl)-propenoyl chloride and pentylamine. M.p. 96.8-97.4°C. Identified by $^1$H-NMR: 0.9-1.0 (t, 3H); 1.3-1.5 (m, 6H); 3.2-3.5 (q, 2H); 6.0-7.6 (m, 7H). (6).

## EXAMPLE 3

3-ethoxycarbonyl-4-(4-fluorophenyl)-1-pentyl-piperidine-2,6-dione

6 g (0.26 mol) sodium was dissolved in 100 ml abs. ethanol. 41.6 g (0.26 mol) diethylmalonate was added with another 200 ml abs. EtOH. 61.9 g (0.20 mol) (3) was added and the mixture refluxed for 4 h. The reaction mixture was very viscous. After 2-3 h it was less viscous. The reaction was followed by means of HPLC.

After standing at 60°C over night, dry toluene was added, then the ethanol was distilled off. The reaction mixture was heated for 24 h at 130°C, resulting in precipitation of the product. The precipitate was dissolved in 1N HCl and extracted with ether. The ether layer was dried yielding 74.5 g brown oil. The product was chromatografed on silicagel (eluent $CH_2Cl_2$/MeOH, 9:1). The product was identified by $^1$H-NMR. Yield: 59.8 g yellow oil. $^1$H-NMR: 0.8-1.3 (m, 12H); 2.8-4.2 (m, 8H); 6.8-7.5 (m, 4H). (7).

1-butyl-3-ethoxycarbonyl-4-phenyl-piperidine-2,6-dione was prepared in exactly the same way from sodium diethylmalonate and (4). The 2,6-dione was an oil. $^1$H-NMR: 0.8-1.3 (m, 10H); 2.8-4.3 (m, 8H); 7.12 (p, 5H). (8).

3-ethoxycarbonyl-4-phenyl-1-pentyl-piperidine-2,6-dione was prepared in exactly the same way from sodium diethylmalonate and (5). The 2,6-dione was an oil. $^1$H-NMR: 0.9-1.4 (m, 12H); 2.9-4.4 (m, 8H); 7.3 (s, 5H). (9).

3-ethoxycarbonyl-4-(3,4-methylenedioxyphenyl)-1-pentyl-piperidine-2,6-dione was prepared in the same way from sodium diethylmalonate and (6). The 2,6-dione which was an oil was identified by $^1$H-NMR (10).

## EXAMPLE 4

(+-)trans-4-(4-fluorophenyl)-3-hydroxymethyl-1-pentylpiperidine

6 g (0.158 mol) $LiAlH_4$ was mixed with dry THF. (7) 7,84 g (0,022 mol) dissolved in dry THF was added under $N_2$. After the addition the reaction mixture was refluxed for 2 h. Subsequently $H_2O$ was dropped very cautiously into the solution. The $H_2O$-phase was extracted with ether. The organic layer was dried over $MgSO_4$ and evaporated to dryness. Yield: 6.3 g. Recrystallized in ether to give 4 g colourless crystals. M.p. 131-132°C. (11).

(+-)trans-1-butyl-3-hydroxymethyl-4-phenyl-piperidine was prepared in the same way. M.p. 110°C. (12).

(+-)trans-3-hydroxymethyl-1-pentyl-4-phenyl-piperidine was prepared in the same way. M.p. 115.1°C. (13).

(+-)trans-4-(3,4-methylenedioxyphenyl)-3-hydroxymethyl-1-pentyl-piperidine was prepared in the same way. M.p. 126-127°C. $^1$H-NMR: 0.9-1.2 (t, 3H); 1.3-2.5 (m, 14H); 3.0-3.5 (m, 5H); 5.8 (s, 2H); 6.6 (s, 3H). (14).

## EXAMPLE 5

(+-)trans-3-benzenesulfonylmethyl-4-(4-fluorophenyl)-1-pentyl-piperidine

10 g (0.036 mol) (11) was dissolved in toluene and MIBC. 10.1 g (0.057 mol) benzenesulfonyl chloride and 8 ml 50% NaOH was added. Reaction time 4 h at room temp. 4N NaOH was added and the organic phase was separated. The water phase was washed with ether. The organic layer was washed with 4N NaOH, then dried and evaporated, resulting in 7 g as an oil. The identity was confirmed by $^1$H-NMR. Yield: 85%. (15).

(+-)-trans-3-benzenesulfonylmethyl-1-butyl-4-phenyl-piperidine was prepared in the same way. Yielding an oil. (16).

(+-)-trans-3-benzenesulfonylmethyl-1-pentyl-4-phenyl-piperidine was prepared in the same way. Yielding an oil. (17).

(+-)trans-3-benzenesulfonylmethyl-4-(3,4-methylenedioxyphenyl)-1-pentyl-piperidine was prepared in the same way. Yielding an oil. (18).

## EXAMPLE 6

(+-)trans-4-(4-fluorophenyl)-3-(3,4-methylenedioxyphenoxymethyl)-1-pentyl-piperidine

7 g (0.017 mol) (15) was dissolved in toluene and MIBC. 2.4 g (0.017 mol) sesamol and 4 ml 50% NaOH was added. The reaction mixture was refluxed for 5 h. The organic layer was washed with 4N NaOH, dried over $MgSO_4$ and evaporated. HPLC showed that 84% of the resulting oil was VI. The oil was dissolved in acetone and 1 eq of oxalic acid was added. Recrystallization of the resulting precipitate gave 3 g of the title compound as the oxalate. This oxalate gave 2.6 g free amine as an oil, by extraction with 4N NaOH/ether.

The hydrochloride was prepared from the free amine and conc. HCl in acetone/ether solution. M.p. 139-139.8°C. (19).

(+-)trans-1-butyl-3-(3,4-methylenedioxyphenoxymethyl)-4-phenyl-piperidine was prepared in the same way. M.p. of the oxalate 115.8°C. (20).

(+-)trans-3-(3,4-methylenedioxyphenoxymethyl)-1-pentyl-4-phenyl-piperidine was prepared in the same way. M.p. of the hydrochloride 132-133°C. (21).

(+-)trans-4-(3,4-methylenedioxyphenyl)-3-(3,4-methylenedioxyphenylmethyl)-1-pentyl-piperidine was prepared in the same way. M.p. of the hydrochloride 156-157°C. (22).

## EXAMPLE 7

(+-)trans-4-(4-fluorophenyl)-3-(3,4-methylenedioxyphenoxymethyl)-piperidine

1.48 g (0.0037 mol) (19) was dissolved in dry 1,2-dichloroethane. 0,8 g (0,0056 mol) 1-chloroethylchloroformate was added slowly under $N_2$ and with rapid stirring at 0°C. The temperature was kept at 0°C for 15 min. Then the reaction mixture was allowed to warm to room temp., and subsequently refluxed for 30 min. Then the volume was reduced to one third, by evaporation. Methanol was added and the mixture refluxed for 1.5 h. The reaction mixture was evaporated in vacuo, giving an oil. The oil was chromatographed on a silica column with $CH_2Cl_2$/methanol (9:1) as eluent, giving 0.25 g of the starting material and 1.05 g of the hydrochloride of the title compound. M.p. 98-98.5°C. (23).

EXAMPLE 8

(-)trans-1-butyl-3-hydroxymethyl-4-phenyl-piperidine-(-)-di-p-toluoyl tartrate

(+-)trans-1-butyl-3-hydroxymethyl-4-phenylpiperidine (4 g) was dissolved in acetone (50 ml) and water (15 ml). (-)-di-p-toluoyl tartaric acid (6.5 g) dissolved in acetone (50 ml) was added at 25°C. The solution was stirred, cooled at 0-5°C. The crystals were filtered off, washed with acetone and dried. Yield 3.5 g, m.p. 177-178°C, $[\alpha]_D^{20}$ = -89.4° (c = 2% in methanol). (24).

(-)trans-3-hydroxymethyl-1-pentyl-4-phenyl-piperidine-(-)-di-p-toluoyl tartrate was prepared in the same way. M.p. 165-166°C, $[\alpha]_D^{20}$ = -86.6° (c = 2% in methanol). (25).

EXAMPLE 9

(-)trans-1-butyl-3-hydroxymethyl-4-phenyl-piperidine

(-)trans-1-butyl-3-hydroxymethyl-4-phenyl-piperidine-(-)-di-p-toluyl tartrate (2 g) was dispensed in water (10 ml), sodium hydroxide 4N (5 ml) and toluene (50 ml) was added. The toluene extract was dried over potassium carbonate, filtered, evaporated at reduced pressure and crystallized from acetone. Yield 0.7 g, m.p. 97-99°C, $[\alpha]_D^{20}$ -27.9° (c = 5% in methanol). (26).

(-)trans-3-hydroxymethyl-1-pentyl-4-phenyl-piperidine was prepared in the same way. M.p. 84-86°C, $[\alpha]_D^{20}$ = -24.2° (c = 5% in methanol). (27).

## Claims

1. A compound having the formula

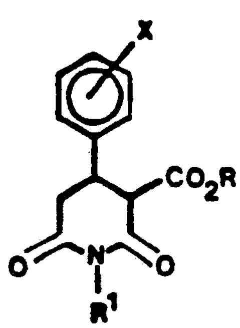

wherein R is alkyl;
$R^1$ is $C_4$-$C_8$-alkyl, $C_{1-8}$-alkoxy-$C_{4-8}$-alkyl, $C_{3-7}$-cycloalkyl, aryloxy-$C_{3-8}$-alkyl, $C_{4-8}$ alkenyl, or $C_{3-8}$-

cycloalkylalkyl; X is hydrogen or halogen.

2. A process for the preparation of a compound having the formula

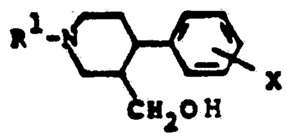

wherein $R^1$ and X have the meanings set forth in claim 1 CHARACTERIZED in reducing a compound having the formula

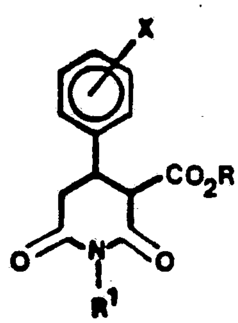

wherein $R^1$, X and R have the meanings set forth above.